# EUROPÄISCHE PATENTANMELDUNG

(11) **EP 2 085 384 A1**
(43) Veröffentlichungstag der Anmeldung: **05.08.2009**
(21) Anmeldenummer: 08170558.4
(22) Anmeldetag: 03.12.2008
(51) Int. Cl.: C07D 211/58

(54) **Verfahren zur Herstellung von 4-Amino-2,2,6,6-tetramethylpiperidin**

(30) Priorität: 01.02.2008 DE 102008000214; 01.07.2008 DE 102008040045
(71) Anmelder: Evonik Degussa GmbH, 45128 Essen (DE)
(72) Erfinder: Brehme, Volker, 48301 Nottuln-Appelhülsen (DE); Dembkowski, Daniel, 45128 Essen (DE)

(57) **Zusammenfassung**

Verfahren zur Herstellung von 4-Amino-2,2,6,6-tetramethylpiperidin durch Umsetzung von 2,2,6,6-Tetramethylpiperidin-4-on mit Ammoniak und Wasserstoff in Gegenwart von Nickel- oder Kobaltkatalysatoren und Wasser, **dadurch gekennzeichnet, dass** die Hauptreaktion bei einem Druck von maximal 50 bar und einer Temperatur von maximal 120 °C bis zu einem Umsatz des 2,2,6,6-Tetramethylpiperidin-4-ons von mindestens 80 % durchgeführt wird und anschließend eine Nachreaktion bei einer höheren Temperatur und bei einem höherem Druck im Vergleich zu dem Druck und Temperatur der Hauptreaktion erfolgt.

## Beschreibung

Die vorliegende Erfindung beschreibt ein Verfahren zur Herstellung von 4-Amino-2,2,6,6-tetramethylpiperidin (TAD) aus 2,2,6,6-Tetramethylpiperidin-4-on (Triacetonamin, TAA), Ammoniak und Wasserstoff in Gegenwart eines Katalysators.

4-Amino-2,2,6,6-tetramethylpiperidin wird aufgrund der 2,2,6,6-Substitution als sterisch gehindert bezeichnet und kann vielfältig eingesetzt werden, insbesondere als Zwischenprodukt bei der Herstellung von UV-Stabilisatoren für Polymere. Wichtig ist hierbei, dass das 4-Amino-2,2,6,6-tetramethylpiperidin neben einer hohen chemischen Reinheit keine bzw. eine möglichst geringe Eigenfärbung besitzt. Auch über eine längere Lagerzeit von mehreren Monaten sollte keine Verfärbung des 4-Amino-2,2,6,6-tetramethylpiperidins stattfinden. Dies ist vor allem dann entscheidend, wenn das 4-Amino-2,2,6,6-tetramethylpiperidin zur Herstellung von Stabilisatoren oder direkt als Additiv verwendet wird, da die Produktqualität des 4-Amino-2,2,6,6-tetramethylpiperidin entscheidenden Einfluss auf die Qualität der stabilisierten Polymere hat.

Die Herstellung von 4-Amino-2,2,6,6-tetramethylpiperidin erfolgt in der Regel durch eine reduktive Aminierung von 2,2,6,6-Tetramethylpiperidin-4-on in ein oder zwei Stufen in Gegenwart von Katalysatoren.

So beschreibt die EP 0 776 887 A1 ein kontinuierliches Verfahren bei Drücken von 285 bis 300 bar in Gegenwart von Metallkatalysatoren, ausgewählt aus Kobalt, Nickel, Ruthenium, Palladium und Platin, und in Abwesenheit von Lösemitteln. Ebenfalls bei Drücken oberhalb von 200 bar und ohne Lösemittel wird das Verfahren gemäß DE 30 03 843 A1 durchgeführt, das auch als diskontinuierliches Verfahren durchgeführt werden kann.

Diskontinuierliche Verfahren werden jedoch in der Regel bei niederem Druck und in Gegenwart von Katalysatoren, die Kobalt oder Nickel aufweisen, durchgeführt. So beschreibt die EP 0 714 890 A2 ein Verfahren bei einem Druck 95 bar und ebenfalls ohne Lösemittel. Hierbei wird das 4-Amino-2,2,6,6-tetramethylpiperidin in einer Reinheit von 95 bis 97 % erhalten.

Häufig werden jedoch Verfahren beschrieben, die in Gegenwart von Lösemitteln, wie beispielsweise Wasser oder Alkoholen, durchgeführt werden. So beschreiben GB 2 176 473 und CN 1358713 A ein Verfahren unter Verwendung von Wasser als Lösemittel und in Gegenwart von Alkali- und Erdalkalimetallen als Cokatalyator bei einem Druck von 10 bis 30 bar. Die erzielten Ausbeuten liegen bei GB 2 176 473 bei 90 bis 95 % und bei CN 1358713 A bei 96,6 %.

Den Einfluss einiger Verfahrensparameter auf das Verfahren zur Herstellung von 4-Amino-2,2,6,6-tetramethylpiperidin beschreiben Li Yang et al. in Chemical Industry and Engineering Vol. 23 No. 4, 323 - 327. So beschreiben Li Yang et al., dass durch den Einsatz von Kobalt- statt Nickel-Katalysatoren sich die Selektivität erhöhen lässt. Die geeignete Temperatur liegt bei 90 bis 100 °C und der Wasserstoffdruck sollte 15 bis 25 bar betragen. Möglichkeiten die Farbstabilität des 4-Amino-2,2,6,6-tetramethylpiperidins zu verbessern, werden wie auch in dem voran beschriebenen Stand der Technik nicht beschrieben.

Hingegen beschreibt die WO 99/16749 ein Verfahren zur Reinigung von 2,2,6,6-tetrasubstituierten 4-Aminopiperidinen, um die Farbstabilität dieser Verbindungen zu verbessern. Nach der Destillation des 2,2,6,6-tetrasubstituierten 4-Aminopiperidins wird dieses in Gegenwart eines Hydrier- oder Dehydrierkatalysators mit Wasserstoff umgesetzt und aus dem Reaktionsgemisch abgetrennt. Mittels diesem Reinigungsschritt kann die APHA-Farbzahl auf kleiner 10 gesenkt werden.

Auch die WO 97/46529 beschreibt ein Verfahren zur Reinigung dieser Piperidine. Hierbei werden zunächst Wasser und hochsiedende Verbindungen aus dem Reaktionsgemisch destillativ entfernt, ein Reduktionsmittel zugesetzt und abschließend das Piperidin durch Destillation isoliert. Durch den Zusatz eines Reduktionsmittels, insbesondere von NaBH₄, kann die APHA-Farbzahl auf kleiner 15 gesenkt werden.

Die beiden PCT-Veröffentlichungen beschreiben jeweils einen zusätzlichen Reinigungsschritt um die gewünschte Farbstabilität zu erzielen.

Es war daher die Aufgabe der vorliegenden Erfindung, ein Verfahren bei Mitteldruck zur Herstellung von 4-Amino-2,2,6,6-tetramethylpiperidin zur Verfügung zu stellen, das die Herstellung von farbstabilem 4-Amino-2,2,6,6-tetramethylpiperidin mit einem APHA-Wert < 20 bei einer Lagerzeit von 6 Monaten ermöglicht.

Überraschenderweise wurde ein Verfahren zur Herstellung von 4-Amino-2,2,6,6-tetramethylpiperidin mit einer hohen Farbstabilität gefunden, das sich dadurch auszeichnet, dass nach der Hauptreaktion eine Nachreaktion bei einer höheren Temperatur und bei einem höherem Druck im Vergleich zu dem Druck und Temperatur der Hauptreaktion erfolgt. Auf diese Weise lässt sich überraschenderweise 4-Amino-2,2,6,6-tetramethylpiperidin mit einer hohen Farbstabilität herstellen, die auch nach einer Lagerzeit von sechs Monaten bei Raumtemperatur erhalten bleibt. Gegenüber dem Stand der Technik hat dieses Verfahren den Vorteil, dass die nachgeschaltete Nachreaktion in demselben Reaktor ohne dazwischen geschaltete Verfahrensschritte erfolgen kann. Ferner müssen bei dieser Nachreaktion keine zusätzlichen Additive, beispielsweise Reduktionsmittel wie NaBH₄, zugesetzt werden. Auf diese Weise kann die Hauptreaktion bei überraschend milden Bedingungen durchgeführt werden und die Bildung von 4-Hydroxy-2,2,6,6-tetramethylpiperdin zurückgedrängt werden. Da die Nachreaktion ohne weitere Zugabe von Additiven und/oder vorgeschaltenen Verfahrensschritten durchgeführt werden kann, stellt dieses Verfahren eine wirtschaftlich interessante Verfahrensvariante bei Mitteldruck dar, um 4-Amino-2,2,6,6-tetramethylpiperidin mit einer hohen Farbstabilität herzustellen. Gegenstand der Erfindung ist somit ein Verfahren zur Herstellung von 4-Amino-2,2,6,6-tetramethylpiperidin durch Umsetzung von 2,2,6,6-Tetramethylpiperidin-4-on mit Ammoniak und Wasserstoff in Gegenwart von Nickel- oder Kobaltkatalysatoren und Wasser, dadurch gekennzeichnet, dass die Hauptreaktion bei einem Druck von maximal 50 bar und einer Temperatur von maximal 120°C bis zu einem Umsatz des 2,2,6,6-Tetramethylpiperidin-4-ons von mindestens 80 % durchgeführt wird und anschließend eine Nachreaktion bei einer höheren Temperatur und bei einem höherem Druck im Vergleich zu dem Druck und Temperatur der Hauptreaktion erfolgt.

Die Messung der Farbstabilität des gemäß dem erfindungsgemäßen Verfahren hergestellten 4-Amino-2,2,6,6-tetramethylpiperidins wird durch Ermittlung der APHA-Farbzahl gemäß EN ISO 6271 durchgeführt. Die APHA-Farbzahl wird anhand einer 20 Gew.-%igen ethanolischen Lösung gemessen. Die Bestimmung der APHA-Farbzahl wird zum einem direkt nach der Aufarbeitung des Reaktionsgemisches, insbesondere nach der Destillation, und nach einer Lagerzeit des 4-Amino-2,2,6,6-tetramethylpiperidins von 30 Tagen bzw. 6 Monaten durchgeführt. Die Lagerung der Proben erfolgt bei Raumtemperatur, Atmosphärendruck und Luftatmosphäre unter normalen Lichtverhältnissen.

Als Edukte werden 2,2,6,6-Tetramethylpiperidin-4-on (TAA), Ammoniak und Wasserstoff in dem erfindungsgemäßen Verfahren eingesetzt. Das Ammoniak kann dem erfindungsgemäßen Verfahren flüssig oder als wässrige Lösung zugeführt werden. Diese wässrige Lösung von Ammoniak hat vorzugsweise einen Gehalt an Ammoniak von 20 bis 50 Gew.-%. Bevorzugt erfolgt die Zugabe des Ammoniaks jedoch in flüssiger Form.

Als Katalysator können in dem erfindungsgemäßen Verfahren Träger- oder Skelettkatalysatoren mit den aktiven Katalysatormetallen Kobalt und/oder Nickel eingesetzt werden. Als Trägermaterialien für die Trägerkatalysatoren können beispielsweise Aluminiumoxid mit einer spezifischen Oberfläche von 100 bis 350 m²/g, Silikate mit einer spezifischen Oberfläche von 400 bis 800 m²/g, Aluminiumsilikate mit einer spezifischen Oberfläche von 200 bis 600 m²/g, Kieselgur mit einer spezifischen Oberfläche von 2 bis 35 m²/g, Aktivkohle mit einer spezifischen Oberfläche von 800 bis 1200 m²/g, Nickel- oder Kobaltoxid mit einer spezifischen Oberfläche von 400 bis 900 m²/g oder Zeolithe mit einer spezifischen Oberfläche von 400 bis 900 m²/g eingesetzt werden. In dem erfindungsgemäßen Verfahren werden bevorzugt Trägerkatalysatoren eingesetzt, die von 10 bis 60 Gew.-% an dem aktiven Katalysatormetall Kobalt und/oder Nickel aufweisen. Diese Trägerkatalysatoren werden vorzugsweise von 1 bis 15 Gew.-%, bevorzugt von 2 bis 12 Gew.-% und besonders bevorzugt von 2,5 bis 10 Gew.-% bezogen auf das eingesetzte 2,2,6,6-Tetramethylpiperidin-4-on in dem erfindungsgemäßen Verfahren eingesetzt.

Bevorzugt werden als Skelettkatalysatoren Katalysatoren vom Typ Raney mit den Aktivmetallen Kobalt und/oder Nickel in dem erfindungsgemäßen Verfahren eingesetzt.

Insbesondere werden Katalysatoren vom Typ Raney eingesetzt, zu deren Herstellung Metalllegierungen eingesetzt wurden, die einen Gehalt von 30 bis 60 Gew.-% an Nickel und/oder Kobalt und von 70 bis 40 Gew.-% an Aluminium aufweisen. Durch Herauslösen des Aluminiums kann ein aktiver Katalysator erzeugt werden, wobei der Rest-Aluminiumgehalt vorzugsweise im Bereich von 2 bis 20 Gew.-% und bevorzugt im Bereich von 5 bis 10 Gew.-% liegt. Der auf diese Weise hergestellte Katalysator vom Typ Raney wird vorzugsweise von 0,5 bis 15 Gew.-%, bevorzugt von 1 bis 12 Gew.-% und besonders bevorzugt von 1,5 bis 10 Gew.-% bezogen auf das eingesetzte 2,2,6,6-Tetramethylpiperidin-4-on in dem erfindungsgemäßen Verfahren eingesetzt.

Die reduktive Aminierung in dem erfindungsgemäßen Verfahren wird in zwei Teilverfahrensschritten durchgeführt, zunächst wird bei relativ milden Bedingungen die Hauptreaktion durchgeführt. Bei einem Umsatz an 2,2,6,6-Tetramethylpiperidin-4-on von mindestens 80 %, bevorzugt bei einem Umsatz von mindestens 90 %, werden Druck und Temperatur erhöht und eine Nachreaktion der reduktiven Aminierung durchgeführt.

Die Reaktionstemperatur der Hauptreaktion wird in dem erfindungsgemäßen Verfahren so gewählt, dass diese niedriger ist als die Reaktionstemperatur der Nachreaktion. Vorzugsweise wird die Hauptreaktion des erfindungsgemäßen Verfahrens bei maximal 120 °C, bevorzugt von 40 bis 110 °C und besonders bevorzugt von 45 bis 100 °C durchgeführt.

Der Druck bei der Hauptreaktion des erfindungsgemäßen Verfahrens wird so gewählt, dass dieser Druck niedriger ist als der Druck bei der Nachreaktion. Vorteilhaft ist hierbei ein Druck von 5 bis 50 bar. Bei der Verwendung von einem Katalysator mit dem Aktivmetall Nickel wird bevorzugt ein Druck von 10 bis 30 bar, besonders bevorzugt von 15 bis 25 bar eingestellt. Wird jedoch ein Katalysator mit dem Aktivmetall Kobalt in dem erfindungsgemäßen Verfahren eingesetzt, so wird die Hauptreaktion bevorzugt bei einem Druck von 15 bis 50 bar und besonders bevorzugt von 20 bis 45 bar durchgeführt. Der für das erfindungsgemäße Verfahren benötigte Druck wird vorzugsweise ausschließlich durch Wasserstoffdruck in dem erfindungsgemäßen Verfahren erzeugt.

Als Lösemittel wird in dem erfindungsgemäßen Verfahren Wasser eingesetzt. Das Wasser kann zum einem als Lösemittel des Ammoniaks in die Reaktionsmischung eingetragen werden, zum anderen kann es auch als Reinsubstanz der Reaktionsmischung zugefügt werden. Durch die Zugabe des Wassers als Reinsubstanz ist es möglich die Mengenverhältnisse von Wasser, Ammoniak und 2,2,6,6-Tetramethylpiperidin-4-on gezielt einzustellen.

Das molare Verhältnis von Wasser zu 2,2,6,6-Tetramethylpiperidin-4-on beträgt in dem erfindungsgemäßen Verfahren vorzugsweise von 2:1 bis 10:1, bevorzugt von 2,5:1 bis 9:1 und besonders bevorzugt von 3:1 bis 7:1. Während das molare Verhältnis von Ammoniak zu 2,2,6,6-Tetramethylpiperidin-4-on vorzugsweise von 1:1 bis 5:1, bevorzugt von 1,5:1 bis 4:1 und besonders bevorzugt von 2:1 bis 3:1 in dem erfindungsgemäßen Verfahren beträgt.

Die Nachreaktion des erfindungsgemäßen Verfahrens wird vorzugsweise bei Temperaturen von mindestens 125 °C, bevorzugt bei Temperaturen von 130 °C bis 200 °C und besonders bevorzugt von 140 °C bis 180 °C durchgeführt.

Der Druck bei der Nachreaktion in dem erfindungsgemäßen Verfahren beträgt vorzugsweise mindestens 30 bar, bevorzugt von 35 bis 150 bar und besonders bevorzugt von 40 bis 100 bar.

Die Nachreaktion wird in dem erfindungsgemäßen Verfahren vorzugsweise in demselben Reaktor wie die Hauptreaktion durchgeführt. Eine Aufarbeitung der Reaktionsmischung nach der Hauptreaktion ist vor der Nachreaktion nicht notwendig.

Nach der Nachreaktion wird in dem erfindungsgemäßen Verfahren vorzugsweise der Reaktor entspannt und zunächst der Katalysator von dem Reaktionsgemisch abgetrennt. Die Abtrennung des Katalysators kann nach bekannten Verfahren gemäß dem Stand der Technik, wie beispielsweise durch Filtration, erfolgen. Bevorzugt wird jedoch bei dem erfindungsgemäßen Verfahren der Reaktionsmischung bzw. dem Reaktoraustrag ein Agglomerationshilfsmittel, wie beispielsweise Toluol, zugegeben, Durch die Zugabe des Agglomerationshilfsmittels zur Reaktionsmischung verbessert sich das Absetzverhalten des Katalysators deutlich, indem Feinanteile des Katalysators agglomerieren und sich wesentlich schneller absetzen. Der Katalysator kann somit einfach von der flüssigen Phase, vorzugsweise durch Dekantieren, abgetrennt werden. Ein Vorteil des Zusatzes bereits kleinster Mengen eines Agglomerationshilfsmittels ist die Verbesserung des Absetzverhaltens des Katalysators aus der Reaktionsmischung, wodurch ein aufwendiger Filtrationsschritt eingespart wird. Hierbei ist unerheblich, ob diese verbesserte Sedimentation aufgrund einer Verringerung der Oberflächenspannung, Grenzflächenspannung, Dichte, Viskosität oder eines anderen Parameters erfolgt. Diese vereinfachte Abtrennung des Katalysators bietet auch einen sicherheitstechnischen Vorteil, da ein abfiltrierter trockener Kobalt- oder Nickelkatalysator selbstentzündlich ist. Bei dem erfindungsgemäßen Verfahren wird vorzugsweise die flüssige Phase, die das Rohprodukt enthält, abdekantiert.

Der aus dem Reaktionsgemisch entfernte Katalysator kann bei dem erfindungsgemäßen Verfahren in einer weiteren reduktiven Aminierung gemäß dem erfindungsgemäßen Verfahrens eingesetzt werden. Hierbei wird der Katalysator als Suspension, bevorzugt als nicht selbstentzündliche Suspension, besonders bevorzugt als nicht-selbstentzündliche wässrige Suspension, der weiteren reduktiven Aminierung zugeführt.

Insbesondere bei der Verwendung von Katalysatoren mit dem Aktivmetall Kobalt erhält man auch bei einem Recycling des Katalysators die gewünschte Farbstabilität des 4-Amino-2,2,6,6-tetramethylpiperidins. Um die Farbstabilität weiter zu verbessern, kann der Katalysator nach dem Einsatz und vor dem nächsten Einsatz aufgearbeitet werden, beispielsweise durch Reinigung mit einem geeigneten Lösemittel, wie beispielsweise Wasser oder niederen Alkoholen, wie Ethanol, und einer anschließenden Wasserstoffbehandlung. Auch wäre es denkbar, 1 bis 30 Gew.-% des Katalysators durch frischen Katalysator auszutauschen.

Die Aufarbeitung der flüssigen Phase, die das Rohprodukt enthält, - nach der Abtrennung des Katalysators - erfolgt in dem erfindungsgemäßen Verfahren vorzugsweise durch den Zusatz von Hilfsstoffen, wie beispielsweise Alkalimetallhydroxiden. Somit kann die Ausbildung von zwei Phasen verbessert werden. Die organische Phase kann nach der Phasentrennung destillativ aufgearbeitet werden.

Bei einer bevorzugten Ausführungsform des erfindungsgemäßen Verfahren wird zu der flüssigen Phase, wobei der Katalysator bereits abgetrennt ist, ein Schleppmittel zugesetzt, das mit Wasser ein Azeotrop bildet, und anschließend wird eine Azeotropdestillation durchgeführt. Vorteilhaft sind hierbei Schleppmittel, deren Azeotrop mit Wasser unterhalb der Siedetemperatur des 4-Amino-2,2,6,6-tetramethylpiperidins und/oder unterhalb der Siedetemperatur von Wasser siedet. Bevorzugt sind dies Verbindungen, die mit Wasser ein binäres Azeotrop bilden und besonders bevorzugt diejenigen, die ein binäres, heterogenes Azeotrop bilden und somit Wasser durch einfache Phasentrennung des Destillats entfernt werden kann. Als Schleppmittel bei der Azeotropdestillation können Kohlenwasserstoffe, wie beispielsweise Hexan, Cyclohexan, Methylcyclohexan, Benzol, Toluol, Xylol, oder Alkohole, wie beispielsweise n-Butanol, 2-Ethylhexanol, Isononanol, eingesetzt werden. Natürlich lassen sich auch alle weiteren, dem Fachmann bekannten Schleppmittel einsetzen.

Durch den Einsatz eines Schleppmittels kann das Wasser, welches bei der Reaktion als Lösungsmittel eingesetzt wird und auch bei der Reaktion entsteht, unter milden Bedingungen abgetrennt werden. Hierdurch wird das 4-Amino-2,2,6,6-tetramethylpiperidin thermisch weniger belastet, woraus eine bessere Farbstabilität und geringere Färbung des Reinproduktes resultiert. Nach der Azeotropdestillation kann eine Destillation des 4-Amino-2,2,6,6-tetramethylpiperidins vorzugsweise unter vermindertem Druck angeschlossen werden.

Die nachfolgenden Beispiele sollen das erfindungsgemäße Verfahren zur Herstellung von 4-Amino-2,2,6,6-tetramethylpiperidin näher erläutern, ohne dass die Erfindung auf diese Ausführungsform beschränkt sein soll.

### Beispiele 1 - 12:

In einem 1 I-Rührautoklaven mit einem Flügelrührer, einer elektrischen Beheizung, Luftkühlung und einem Wasserstoff-Massendurchflussregler werden 300 g 2,2,6,6-Tetramethylpiperidin-4-on, deionisiertes Wasser und 11 g des Katalysators unter Argon vorgelegt. Als Katalysatoren wurden zum einen B113W (Aktivmetall Nickel) und zum anderen B2112Z (Aktivmetall Kobalt) der Fa. Evonik Degussa GmbH eingesetzt. Anschließend wird der Autoklav dreimal mit Stickstoff gespült. Der Rührer wird auf eine Umdrehungszahl von 300 rpm eingestellt. Nun werden 83 g flüssiges Ammoniak zudosiert. Anschließend wird mit Hilfe von Wasserstoff der Druck auf den gewünschten Druck der Hauptreaktion und ferner die Temperatur eingestellt. Die Nachreaktion erfolgt bei einer Temperatur von 150 °C und einem Druck von 50 bar für ca. 3 Stunden. Davon abweichende Bedingungen für die Nachreaktion sind in der folgenden Tabelle 1 vermerkt. Die Tabelle 1 zeigt sowohl die Versuchsparameter, als auch die Ergebnisse der durchgeführten Beispiele.

### Beispiele 13 - 16:

In einem 2 I-Rührautoklaven mit Blattrührer und einem beheiztem Doppelmantel werden 450 g 2,2,6,6-Tetramethylpiperidin-4-on, 250 g deionisiertes Wasser und 33 g eines Kobalt-Katalysators (Typ: Evonik Degussa GmbH B2112Z, wasserfeucht) unter Argon vorgelegt. Anschließend wird der Autoklav verschlossen und dreimal mit Stickstoff gespült. Der Rührer wird auf eine Drehzahl von 500 UPM eingestellt. Nun werden 125 g flüssiges Ammoniak zudosiert und auf 90°C Innentemperatur aufgeheizt. Anschließend wird mit Hilfe von Wasserstoff ein Druck von 40 bar eingestellt. Die Nachreaktion erfolgt bei 150°C und 50 bar H₂ für ca. eine Stunde. Danach wird abgekühlt und der Autoklav bei ca. 50°C Innentemperatur entspannt, zweimal mit Stickstoff gespült und das Reaktionsprodukt unter Argon abgelassen.

Zur Aufarbeitung wird das Rohprodukt bei 50°C aufgerührt und 10 g Toluol zugegeben. Erhalten wird ein dreiphasiges Rohprodukt (untere Phase (fest) enthält den Kobalt-Katalysator (fest), mittlere Phase (flüssig, hellgelb, leicht trübe) enthält das gewünschte Produkt; obere Phase (flüssig): hellgelbe- klare Toluol-Phase. Die beiden flüssigen Phasen werden mit einem Saugrohr abgetrennt. Der zurückbleibende Katalysator wird mit 100 g deionisiertem Wasser versetzt und im nächsten Ansatz wieder eingesetzt. In dem folgenden Ansatz werden dann anstelle von 250 g deionisiertem Wasser nur noch 150 g deionisiertes Wasser zugegeben.

Die beiden flüssigen Phasen (805 g, gelblich) werden mit weiteren 140 g Toluol in einer Destillationsapparatur mit 10 cm Glaskolonne, Wasserabscheider, Rückflusskühler und Ölbad aufgearbeitet, in dem bei einer Sumpftemperatur von maximal 130°C und einer Kopftemperatur von maximal 110°C eine Azeotropdestillation durchgeführt wird (Destillat (Wasser): 312 g und Sumpf: 580 g). Anschließend wird bei verbessertem Vakuum das restliche Toluol bei einer Sumpftemperatur von maximal 70°C und einem Druck von 150 - 30 mbar abdestilliert. Es werden 450 g gelb gefärbtes 4-Amino-2,2,6,6-tetramethylpiperidin-Rohprodukt im Sumpf zurückbehalten. Als Destillat fallen 130 g Toluol an. Nachdem das 4-Amino-2,2,6,6-tetramethylpiperidin-Rohprodukt noch einmal über Kopf destilliert wurde (50 cm-Kolonne; Sumpftemperatur 80 - 100°C, Kopftemperatur 78°C, Druck: 15 mbar), wird ein farbloses, lagerstabiles reines 4-Amino-2,2,6,6-tetramethylpiperidin erhalten (siehe auch Tabelle 2).

**Tabelle 1:**

| Versuch | Katalysator | Lösemittel | | Hauptreaktion | | | Nachreaktion | APHA (20 %ig in Ethanol) *(nach ...)* | | | Analyse des Reaktionsprodukts mittels GC *(in Flächen-% GC)* | | |
|---|---|---|---|---|---|---|---|---|---|---|---|---|---|
| | | Art | *(in* g) | Druck *(H₂, in bar)* | Temperatur-verlauf *(in °C)* | Dauer *(in h)* | | Destillation | 30 Tagen | 6 Monaten | TAA | TAD | TAA-ol |
| 1 (VB) | Ni | Wasser | 200 | 20 | 90 | 5 | - | 23 | 98 | 97 | 0 | 95,63 | 1,93 |
| 2 (VB) | Ni | Wasser | 200 | 20 | 90 | 16 | - | 0 | 7 | 31 | 0 | 93,80 | 2,64 |
| 3 (B) | Ni | Wasser | 200 | 20 | 90 | 1 | X *(p* = 70 *bar)* | 1 (nach 4 Tagen) | 4 | 3 | 0 | 93,44 | 2,98 |
| 4 (B) | Ni | Wasser | 222 | 40 | 55 - 100 | 3 | X | 2 | 0 | 1 | 0 | 90,73 | 6,54 |
| 5 (B) | Ni | Wasser | 111 | 40 | 55 - 100 | 3 | X | 7 | 13 | 12 | 0 | 92,10 | 5,49 |
| 6 (B) | Ni | Wasser | 111 | 20 | 55 - 100 | 3 | X | 2 | 5 | 3 | 0 | 92,49 | 4,65 |
| 7 (B) | Co | Wasser | 111 | 40 | 80 - 100 | 4 | X | 3 | 5 | 4 | 0 | 94,27 | 2,38 |
| 8 (VB) | Co | Methan ol | 111 | 40 | 100 - 120 | 3 | X | 43 | 29 | 20 | 0 | 94,86 | 1,27 |
| 9 (B) | Co | Wasser | 111 | 20 | 90 - 120 | 3 | X | 1 | 2 | 3 | 0 | 85,05 | 2,89 |
| 10 (B) | Co | Wasser | 111 | 40 | 90 - 130 | 3 | X | 1 | 2 | 2 | 0 | 94,07 | 2,53 |
| 11 (B) | Co (aus 10) | Wasser | 111 | 40 | 90 - 130 | 3 | X | 2 | 7 | 10 | 0 | 92,32 | 3,50 |
| 12 (B) | Co (aus 11) | Wasser | 111 | 40 | 100 - 130 | 4 | X | 9 | 8 | 8 | 0 | 91,95 | 2,82 |

| | | | | | | | | | | | | | |
|---|---|---|---|---|---|---|---|---|---|---|---|---|---|
| B : erfindungsgemäßes Beispiel VB : Vergleichsbeispiel | | | | | | | | | | | | | |

**Tabelle 2:**

| Versuch | Katalysator | Lösemittel | | Hauptreaktion | | | Nachreaktion | APHA (20 %ig in Ethanol) *(nach ...)* | | | Analyse des Reaktionsprodukts mittels GC *(in Flächen-% GC)* | | |
|---|---|---|---|---|---|---|---|---|---|---|---|---|---|
| | | Art | *(in g)* | Druck *(H₂, in bar)* | Temperatur -verlauf *(in °C)* | Dauer *(in h)* | | Destillation | 30 Tagen | 6 Monaten | TAA | TAD | TAA-ol |
| 13 (B) | Co | Wasser | 250 | 40 | 90-120 | 4 | X | 0 | 3 | | 0 | 94,54 | 3,90 |
| 14 (B) | Co (aus 13) | Wasser | 250 | 40 | 90-120 | 5 | X | 1 | 3 | | 0 | 91,78 | 3,76 |
| 15 (B) | Co (aus 14) | Wasser | 250 | 40 | 90-120 | 4 | X | 3 | 3 | | 0 | 92,93 | 3,76 |
| 16 (B) | Co (aus 15) | Wasser | 250 | 40 | 100-120 | 4 | X | 4 | 4 | | 0 | 93,32 | 3,15 |

## Patentansprüche

1. Verfahren zur Herstellung von 4-Amino-2,2,6,6-tetramethylpiperidin durch Umsetzung von 2,2,6,6-Tetramethylpiperidin-4-on mit Ammoniak und Wasserstoff in Gegenwart von Nickel- oder Kobaltkatalysatoren und Wasser,
**dadurch gekennzeichnet,**
**dass** die Hauptreaktion bei einem Druck von maximal 50 bar und einer Temperatur von maximal 120 °C bis zu einem Umsatz des 2,2,6,6-Tetramethylpiperidin-4-ons von mindestens 80 % durchgeführt wird und
anschließend eine Nachreaktion bei einer höheren Temperatur und bei einem höherem Druck im Vergleich zu dem Druck und Temperatur der Hauptreaktion erfolgt.

2. Verfahren gemäß Anspruch 1,
**dadurch gekennzeichnet,**
**dass** Wasser und 2,2,6,6-Tetramethylpiperidin-4-on im molaren Verhältnis von 2,5:1 bis 9:1 eingesetzt werden.

3. Verfahren gemäß Anspruch 2,
**dadurch gekennzeichnet,**
**dass** Wasser und 2,2,6,6-Tetramethylpiperidin-4-on im molaren Verhältnis von 3:1 bis 7:1 eingesetzt werden.

4. Verfahren gemäß zumindest einem der Ansprüche 1 bis 3,
**dadurch gekennzeichnet,**
**dass** Katalysatoren vom Typ Raney mit den Aktivmetallen Kobalt und/oder Nickel eingesetzt werden.

5. Verfahren gemäß Anspruch 4,
**dadurch gekennzeichnet,**
**dass** von 0,5 bis 15 Gew.-% an Katalysator vom Typ Raney bezogen auf das eingesetzte 2,2,6,6-Tetramethylpiperidin-4-on eingesetzt werden.

6. Verfahren gemäß zumindest einem der Ansprüche 1 bis 5,
**dadurch gekennzeichnet,**
**dass** die Hauptreaktion bei einer Temperatur von 40 bis 110 °C durchgeführt wird.

7. Verfahren gemäß zumindest einem der Ansprüche 1 bis 6,
**dadurch gekennzeichnet,**
**dass** die Hauptreaktion bei einem Druck von 5 bis 50 bar durchgeführt wird.

8. Verfahren gemäß zumindest einem der Ansprüche 1 bis 7,
**dadurch gekennzeichnet,**
**dass** die Nachreaktion bei einer Temperatur von 130 bis 200 °C durchgeführt wird.

9. Verfahren gemäß zumindest einem der Ansprüche 1 bis 8,
**dadurch gekennzeichnet,**
**dass** die Nachreaktion bei einem Druck von 35 bis 150 bar durchgeführt wird.

10. Verfahren gemäß zumindest einem der Ansprüche 1 bis 9,
**dadurch gekennzeichnet,**
**dass** die Nachreaktion in demselben Reaktor wie die Hauptreaktion durchgeführt wird.

11. Verfahren gemäß zumindest einem der Ansprüche 1 bis 10,
**dadurch gekennzeichnet,**
**dass** keine Aufarbeitung der Reaktionsmischung nach der Hauptreaktion und vor der Nachreaktion erfolgt.

12. Verfahren gemäß zumindest einem der Ansprüche 1 bis 11,
**dadurch gekennzeichnet,**
**dass** Ammoniak und 2,2,6,6-Tetramethylpiperidin-4-on im molaren Verhältnis von 1,5:1 bis 4:1 eingesetzt werden.

13. Verfahren gemäß zumindest einem der Ansprüche 1 bis 12,
**dadurch gekennzeichnet,**
**dass** der Katalysator von der flüssigen Phase durch Dekantieren abgetrennt wird, wobei vorher der Reaktionsmischung ein Agglomerationshilfsmittel zugegeben wird.

14. Verfahren gemäß Anspruch 13,
**dadurch gekennzeichnet,**
**dass** der Katalysator als Suspension einer weiteren reduktiven Aminierung zugeführt wird.

15. Verfahren gemäß Anspruch 13 bis 14,
**dadurch gekennzeichnet,**
**dass** der flüssigen Phase ein Schleppmittel zugesetzt und anschließend eine Azeotropdestillation durchgeführt wird.
